# EUROPÄISCHE PATENTSCHRIFT

(11) **EP 0 972 036 B1**
(45) Veröffentlichungstag und Bekanntmachung des Hinweises auf die Patenterteilung: **30.08.2006**
(21) Anmeldenummer: 98912225.4
(22) Anmeldetag: 13.02.1998
(51) Int. Cl.: C12N 15/12, C07K 14/47, C07K 16/18, C12N 5/10, A61K 39/00, A61K 48/00, A01K 67/027

(54) **HEMMUNG DER PRION-VERMEHRUNG DURCH DOMINANT-NEGATIVE PRIONPROTEIN-MUTANTEN**
PRION PROPAGATION INHIBITION BY DOMINANT-NEGATIVE PRION PROTEIN MUTANTS
INHIBITION DE LA PROPAGATION D'UN PRION AU MOYEN DE MUTANTS DE PROTEINE DE PRION A EFFET DOMINANT-NEGATIF

(30) Priorität: 14.02.1997 DE 19705786
(43) Veröffentlichungstag der Anmeldung: 19.01.2000
(73) Patentinhaber: Deutsches Krebsforschungszentrum Stiftung des öffentlichen Rechts, 69120 Heidelberg (DE)
(72) Erfinder: HÖLSCHER, Christina, D-69121 Heidelberg (DE); BÜRKLE, Alexander, D-69181 Leimen (DE)
(74) Vertreter: Schüssler, Andrea
(86) Internationale Anmeldenummer: PCT/DE1998/000429
(87) Internationale Veröffentlichungsnummer: WO 1998/036059

(56) Entgegenhaltungen:
- WO-A-96/39834
- MURAMOTO, T. ET AL.: "Recombinant scrapie-like prion protein of 106 amino acids is soluble." PNAS, Bd. 93, Dezember 1996, Seiten 15457-62, XP002074795
- PRIOLA, S.A. ET AL.: "Heterologous PrP molecules interfere with accumulation of protease-resistant PrP in scrapie-infected murine neuroblastoma cells." JOURNAL OF VIROLOGY, Bd. 68, Nr. 8, August 1994, Seiten 4873-8, XP002074796
- WARWICKER, J. ET AL.: "A model for prion protein dimerisation based on alpha-helical packing." BICHEMICAL AND BIOPHYSICAL RESEARCH COMMUNICATIONS, Bd. 226, Nr. 3, September 1996, Seite 777 XP002074797
- GASSET, M. ET AL.: "Predicted alpha-helical regions of the prion protein when synthesized as peptides form amyloid." PNAS, Bd. 89, November 1992, Seiten 10940-4, XP002074798 in der Anmeldung erwähnt
- BALDWIN, M.A. ET AL.: "Prion protein isoforms, a convergence of biological and structural investigations." JOURNAL OF BIOLOGICAL CHEMISTRY, Bd. 270, Nr. 33, 18. August 1995, Seiten 19197-200, XP002074799
- NGUYEN, J. ET AL.: "Prion protein peptides induce alpha-helix to beta-sheet conformation transitions." BIOCHEMISTRY, Bd. 34, 1995, Seiten 4186-92, XP002074800
- HÖLSCHER, C. ET AL.: "Overexpression of nonconvertible PrPc-delta-144-121 in scrapie-infected mouse neuroblastoma cells leads to trans-dominant inhibition of wild-type PrPsc accumulation." JOURNAL OF VIROLOGY, Bd. 72, Nr. 2, 1. Februar 1998, Seiten 1153-9, XP002074801

## Beschreibung

Die vorliegende Erfindung betrifft einen Vektor mit einem Nukleinsäureinsert, das für ein mutiertes Prionprotein kodiert, ein mutiertes Prionprotein, eine ein solches kodierende DNA und ein Verfahren zur Herstellung eines solchen. Ferner betrifft die Erfindung Antikörper, ein Vakzinierungsmittel und die Verwendung dieser vorgenannten Gegenstände. Außerdem betrifft die vorliegende Erfindung nichtmenschliche Säuger, z.B. Nutztiere, die gegen Prioneninfektionen resistent sind.

Die sich in den letzten Jahren ausbreitenden Prionkrankheiten, zu welchen u.a. BSE, die Creutzfeldt-Jakob-Krankheit sowie Scrapie gehören, sind eine Gruppe von absolut tödlich verlaufenden neurodegenerativen Erkrankungen bei Mensch und Tier, die durch infektiöse Erreger verursacht werden, deren Aufbau noch ungeklärt ist und die im folgenden als "Prionen" bezeichnet werden. Für diese Krankheiten gibt es bisher noch keine zufriedenstellenden Möglichkeiten der Prophylaxe bzw. der Therapie. Erschwert wird die Entwicklung von solchen Prophylaxe/Therapiemöglichkeitendadurch, daß die molekulare Pathogenese von Prionkrankheiten zur Zeit noch nicht ausreichend geklärt ist. Mit diversen Pharmaka konnte bisher lediglich-eine geringfügige Verzögerung der Prionreplikation erreicht, keineswegs aber die Infektion zum Stillstand gebracht werden (Priola et al., Inhibition of scrapie-associated PrP accumulation. Probing the role of glycosaminoglycans in amyloidogenesis, Mol. Neurobiol. 8, S. 113-120 (1994)). Außerdem ist eine solche Therapie mit erheblichen Nebenwirkungen verbunden, was dadurch begründet ist, daß diese Medikamente nur sehr unspezifisch angreifen.

Muramoto et al.(PNAS 93, 1996, S. 15457) beschreiben ein mutiertes Prionprotein, bei dem der gesamte Helix1-Bereich (H1-Bereich) der Aminosäuren 108 bis 121, sowie ein Bereich der Aminosäuren 23 bis 88 gegenüber der Ausgangsform deletiert ist.

Baldwin et al.(J.Biol.Chem. 270, 1995, S. 19197); Dohura et al.(Biochem.Biophys.Res.Comm. 163, 1989, S. 974); und Kazmirski et al., (Chem. & Biol. 2, 1995, S. 305) beschreiben alle eine pathogene Punktmutation vom Substitutionstyp an Position 117 im H1-Bereich.

Die Aufgabe der vorliegenden Erfindung besteht deshalb darin, ein Mittel bereitzustellen, um Nutztiere, deren Gewebe oder Produkte für den menschlichen Gebrauch als Nahrungsmittel, Medikamentenbestandteile oder Kosmetikartikel verwendet werden, gegen die Prionvermehrung resistent zu machen, und infizierte menschliche oder tierische Individuen vor dem Angehen der Infektion bzw. deren Fortschreiten zu schützen.

Erfindungsgemäß wird dies durch die Gegenstände in den Patentansprüchen erreicht.

Gegenstand der vorliegenden Erfindung ist somit ein Vektor mit einem Nukleinsäureinsert, das für ein mutiertes Prionprotein kodiert, welches die Fähigkeit zur Konversion in die amyloidartige, krankheitsassozüerte Form des Prionproteins verloren hat und von dem eine Hemmwirkung auf die Konversion von in einem Organismus natürlicherweise vorhandenen endogenen Wildtyp-Prionproteins ausgeht, wobei das mutierte Prionprotein eine Deletion oder eine Substitution von mindestens zwei Aminosäuren zwischen den Aminosäuren 114 und 121, (H1-Bereich) aufweist. Dieser sollte in bevorzugter Weise ein für die Genprophylaxe bzw. Gentherapie geeigneter Vektor sein.

Im Rahmen der Erfindung wurden von den Erfindern kritische Bereiche, z.B. der Helix 1-Bereich (H1)(Gasset et al., Predicted alpha-helical regions of the prion protein when synthesized as peptides form amyloid, Proc. Natl. Acad. Sci. USA 89, S. 10940, 1992), im Prionprotein gentechnisch verändert, indem z.B. Mutationen in Form von Insertionen, Substitutionen oder Deletionen, insbesondere Deletionen durchgeführt wurden. Es wurde von den Erfindern dabei herausgefunden, daß diese veränderten rekombinanten Proteine ihre Fähigkeit verlieren, in die amyloidartige, krankheitsassoziierte Form des Prionproteins (PrP^{Sc}) überführt werden zu können. Außerdem wurde gefunden, daß von der Anwesenheit der mutierten Proteine eine Hemmwirkung auf die Überführung des im Organismus natürlicherweise vorhandenen endogenen Wildtyp-Prionproteins (PrP^{c}) in die krankheitsassoziierte Form (PrP^{sc}) ausgeht (sog. dominant negativer Effekt).

Der Ausdruck "für die Gentherapie geeigneter Vektor" umfaßt jeglichen Vektor, der sich für den Gentransfer, d.h. das Einführen von Nukleinsäuren in Zellen eignet. Der Vektor kann in den Zellen episomal verbleiben oder in das Genom integriert werden. Ferner kann der Vektor ein Plasmid- oder Virus-Vektor sein. Beispiele eines Virus-Vektors sind retrovirale, Herpes Simplex Virus-, Cytome-galievirus- (CMV), Adenovirus-, Vacciniavirus- oder Adeno-assoziierte Virus (AAV)-Vektoren. Als bevorzugte Plasmidvektoren sind pUC18 oder pUC19 zu nennen.

Der Begriff "mutiertes Prionprotein" umfaßt ein Prionprotein, das gegenüber dem Wildtyp mittels geeigneter Maßnahmen, die dem Fachmann hinreichend bekannt sind, in seiner Funktion und/oder seiner Länge verändert worden ist. Dies kann erfindungsgemäβ durch Einbringen von Mutationen auf Nukleinsäureebene geschehen, d.h. Durchführen von Substitutionen und insbesondere Deletionen. Verfahren zur Erzeugung der vorstehenden Änderungen in der Nucleinsäuresequenz sind dem Fachmann bekannt und in Standardwerken der Molekularbiologie beschrieben, beispielsweise in Sambrook et al., Molecular Cloning: A Laboratory Manual, 2. Ausgabe, Cold Spring Harbor Laboratory Press, Cold Spring Harbor NY (1989). Die Mutation wird im H1-Bereich des Prionproteins, innerhalb dessen eine Ansammlung identischer Aminosäuren vorkommt (Ala-Gly-Ala-Ala-Ala-Ala-Gly-Ala), und welcher bei allen Tierarten konserviert ist, durchgeführt. Bezüglich der Sequenzen der Prionproteine verschiedener Organismen sei auf Prusiner, S.B., Molecular structure, biology and genetics of prions, Adv. Virus Res. 35, S. 83 ff. (1988); Schätzl, H.M. et al., Prion protein gene variation among primates, J. Mol. Biol. 245, S. 362 ff. (1995) und Gabriel, J.M. et al., Molecular cloning of a candidate chicken prion protein, Proc. Natl., Acad. Sci. USA 89, S. 9097 ff. (1992) verwiesen. Die Veränderung betrifft die Aminosäuren 114-121. Diese Veränderung muß sinnvollerweise mindestens zwei Aminosauren umspannung da sonst die beschriebenen Eigenschaften wie die Nichtkonvertierbarkeit in die krankheitsassoziierte Form des Prionproteins (PrP^{sc}) und/oder die transdominante Hemmung der Äkkumulation von Wildtyp-PrP^{sc} nicht zu erwarten sind. Da die Region von Aminosäure 114 bis 121 nachgewiesenermaßen die am stärksten amyloidogenen Aminosäuren des Prionproteins enthalten, ist eine Deletion der bevorzugte Weg zur Herstellung eines veränderten Proteins mit den vorgenannten Eigenschaften. Ein anderer Weg ist nach Deletion von mind. zwei der amyloidogenen Aminosäuren in der o.g. Region die Substitution durch Aminosäuren, welche bekanntermaßen keine Neigung haben, β-Faltblattstruktur zu bilden oder sogar eine existierende ß-Faltblattstruktur zerstören können (z.B. Prolin). Eine weitere vorteilhafte Veränderung des Prionproteins erfolgt am Carboxyterminus. Durch diese Veränderung wird die bereits spontan erfolgende Sekretion von PrP-Molekülen verstärkt (Borchelt et al., Release of the cellular prion protein from cultured cells after loss of its glycoinositol phospholipid anchor, Glycobiology 3, S. 319-329 (1993)). Erfindungsgemäß wird dies dadurch erreicht, daß mutierte Prionproteine von den produzierenden Zellen durch Fehlen von Glycoinosit-Phospholipidankern in erhöhtem Maße sezerniert werden. Somit können durch. Diffusion auch solche Zellen in räumlicher Nachbarschaft erreicht werden, welche die mutierten Prionproteine nicht selbst exprimieren. Mithin wird also der gewünschte dominant negative Effekt auf eine viel größere Anzahl von Zielzellen ausgeweitet.

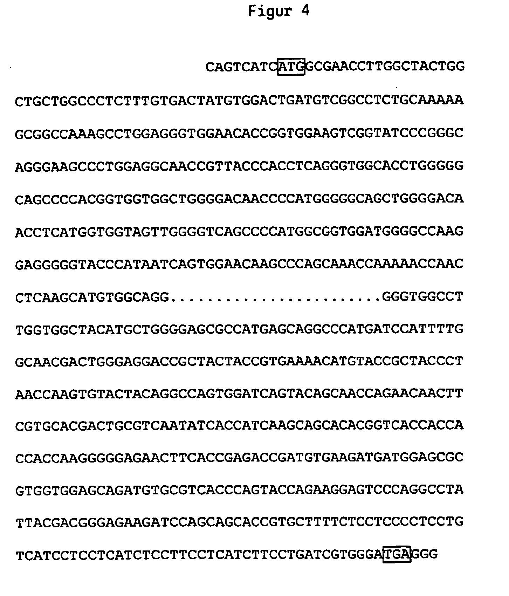

Der Ausdruck "Nukleinsäureinsert" umfaßt jegliche Nukleinsäure, wie DNA oder RNA, die für ein mutiertes Prionprotein kodiert. Günstig ist es, wenn das Nukleinsäureinsert exprimierbar ist, z.B. wenn es unter der Kontrolle eines konstitutiven oder induzierbaren Promotors steht. Bevorzugt ist ein Nukleinsäureinsert in Form einer DNA, die folgendes umfaßt:'
(a) die DNA von Fig. 4 oder eine hiervon durch ein oder mehrere Basenpaare unterschiedliche DNA,
(b) eine mit der DNA von (a) hybridisierende DNA, oder
(c) eine mit der DNA von (a) oder (b) über den degenerierten genetischen Code verwandte DNA.

Der Ausdruck "hybridisierende DNA" weist auf eine DNA hin, die unter üblichen Bedingungen, insbesondere bei 20°C unter dem Schmelzpunkt der DNA, mit einer DNA von (a) hybridisiert. Der Begriff "hybridisieren" bezieht sich dabei auf konventionelle Hybridisierungsbedingungen, vorzugsweise auf Hybridisierungsbedingungen, bei denen als Lösung 5xSSPE, 1% SDS, 1xDenhardts-Lösung verwendet wird und die Hybridisierungstemperaturen zwischen 35°C und 70°C, vorzugsweise bei 65°C liegen. Nach der Hybridisierung wird vorzugsweise zuerst mit 2xSSC, 1 % SDS und danach mit 0,2xSSC bei Temperaturen zwischen 35°C und 70°C, vorzugsweise bei 65°C gewaschen (zur Definition von SSPE,SSC und Denhardts-Lösung siehe Sambrook et al.,supra). Besonders bevorzugt sind stringente Hybridisierungsbedingungen, wie sie beispielsweise in Sambrook et al ., supra, beschrieben sind.

Erfindungsgemäß wird in einen vorstehenden Vektor ein vorstehendes Nukleinsäureinsert eingefügt. Die Einfügung erfolgt derart, daß das Nukleinsäurefragment exprimiert werden kann. Dies kann erreicht werden, indem das Nukleinsäurefragment in eine im Vektor vorhandene Expressionseinheit in Phase inseriert wird. Dazu kann es notwendig sein, eine in der Expressionseinheit vorliegende DNA zumindest teilweise zu entfernen. Auch kann es vorteilhaft sein, Elemente der vorhandenen Expressionseinheit, wie Enhancer, Promotor oder Polyadenylierungsstelle, zumindest teilweise durch andere zu ersetzen. Vorzugsweise wird in eine Expressionseinheit ein Promotor eingeführt, der spezifisch für eine Gewebeart ist, wodurch die Expression des unter der Kontrolle des Promotors stehenden Nukleinsäureinserts gewebespezifisch wird. Die Expression des vorstehenden Nukleinsäureinserts kann ferner in einer Expressionseinheit erfolgen, die hierzu in den Vektor eingeführt werden muß. Um das erfindungsgemäße mutierte Prionprotein zu exprimieren, wird der Vektor in geeignete Zellen transformiert. Beispiele solcher Zellen umfassen die E. coli Stämme HB101, DH1, x1776, JM101, JM109, BL21 und SG 13009, den Hefestamm Saccharomyces cerevisiae und die tierischen Zellen Neuro 2a, 3T3, FM3A, CHO und COS. Desweiteren kennt der Fachmann Bedingungen, transformierte bzw. transfizierte Zellen zu kultivieren. Auch sind ihm Verfahren bekannt, das durch das Nukleinsäureinsert (insbesondere durch die DNA) exprimierte Protein zu isolieren und zu reinigen. Ein solches Protein und Fragmente davon sind somit ebenfalls Gegenstand der vorliegenden Erfindung.

Im Falle von Virus-Vektoren erweist es sich oftmals als günstig, das Nukleinsäurefragment in eine im Vektor vorhandene Expressionseinheit einzufügen. Die hiermit möglicherweise verbundene Entfernung oder Teilentfernung von in der Expressionseinheitvorliegender Virus-DNA führt dann zu einem Virus-Vektor, der in einer Virus-Funktion einen Defekt aufweist. Dieser Defekt kann als Selektionsmarker genutzt werden. Andererseits kann der Defekt, falls notwendig, durch übliche Verfahren, wie Komplementation in trans, ausgeglichen werden. Erfindungsgemäß werden Virus-Vektoren bevorzugt, in denen das Nukleinsäurefragment so eingefügt ist, daß die Virus-Vektoren alleine nicht mehr zur Bildung infektiöser Wildtyp-Viren in der Lage sind.

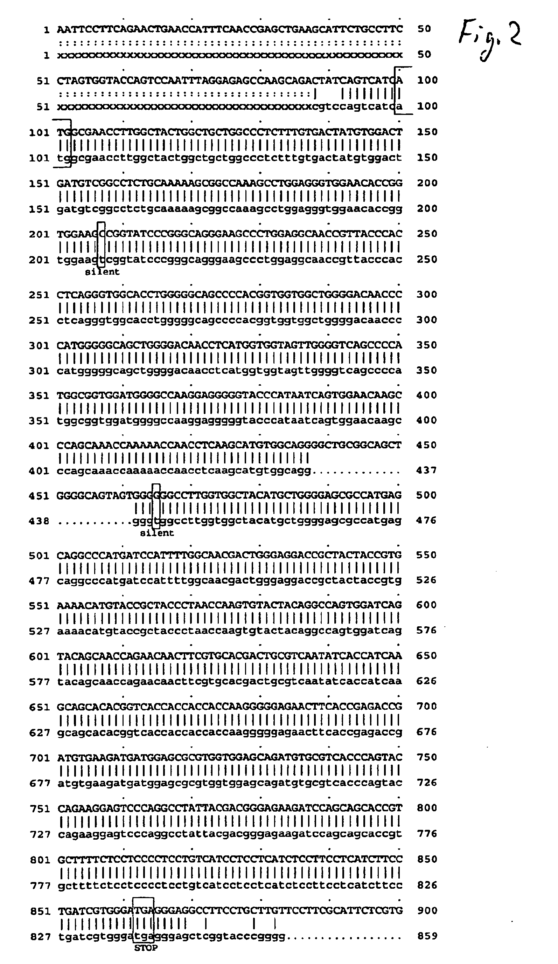

Zur Herstellung eines vorstehenden Vektors können übliche Verfahren durchgeführt werden. In einer bevorzugten Ausführungsform handelt es sich bei dem erfindungsgemäßen Vektor um pUC-PrPΔH1 oder pCMV-PrPΔH1. Letzterer wurde am 6. Februar 1997 bei der DSMZ Braunschweig (Deutsche. Sammlung von Mikroorganismen und Zellkulturen GmbH, Mascheroder Weg 1b, 38124 Braunschweig) unter der Nummer DSM 11400 als E. coli-Kultur hinterlegt. Die Herstellung des Vektors pCMV-PrPΔH1 wird nachfolgend in Beispiel 1 beschrieben. Das dazu verwendete Insert (Nukleinsäurefragment) PrPΔH1 stammt aus dem Maus-Prionproteingen und codiert für ein mutiertes Protein, das gegenüber dem Wildtyp um 8 Aminosäuren verkürzt ist. Die Deletion wurde mittels gerichteter Mutagenese ("site directed mutagenesis") in das Wildtypgen eingebracht und befindet sich von Position 438 bis 461 (basierend auf der Zählung gemäß Locht et al., Proc. Natl. Acad. Sci. USA 83, S. 6372 (1986)), d.h im H 1-Bereich des Prionproteins und betrifft die Aminosäuren (Ala-Ala-Ala-Ala-Gly-Ala-Val-Val [Aminosäuren 114-121]) Ein Sequenzvergleich zwischen dem Maus-Wildtypgen und dem verwendeten Insert PrPΔH1 ist in Fig. 2 gezeigt. Die DNA-Sequenz von PrPΔH1 ist außerdem noch in Fig. 4 gezeigt. Der zweite oben genannte erfindungsgemäße Vektor pUC-PrPΔH1 wurde durch Einklonieren des PrPΔH1-Insert gemäß Fig. 4 in pUC19 üder AatII/SacI hergestellt.

Die vorstehenden Gegenstände eignen sich besonders gut dafür, eine Prophylaxe bzw. Therapie für Prionkrankheiten bereitzustellen. Dabei wird einerseits der Effekt ausgenutzt, daß die mutierten Prionproteine nicht in die infektiöse Form umgewandelt werden können und andererseits, daß durch Überexpression der mutierten Prionproteine vorhandene zelluläre Wildtyp-Prionproteine (PrP^{c}) an der Konversion zu PrP^{sc} gehindert werden. Im letzteren Fall ist es empfehlenswert, die PrP-Mutation an der PrP-DNA-Sequenz der Zielspezies in analoger Weise durchzuführen, um eine möglichst starke Protein-Protein-Wechselwirkung zwischen PrP^{c} und Pr^{psc} zu gewährleisten, die für den gewünschten Hemmeffekt notwendig ist. Die Prophylaxe von Prionkrankheiten geschieht z. B. durch die Herstellung von transgenen Nutztieren, die ein mutiertes PrP-Gen entweder ausschließlich oder zusätzlich zum endogenen Wildtyp-PrP-Gen tragen. Dem Fachmann sind geeignete Verfahren zur Transgenese bekannt. Es stehen beispielsweise effiziente Methoden zur Herstellung transgener Rinder durch Insertion fremder (zusätzlicher) Gene zur Verfügung (Hyttinen et al., "Generation of transgenic dairy cattle from transgene-analyzed and sexed embryos produced in vitro", Biotechnology N.Y., 12, S. 606-608, 1994). Desweiteren sind der Mechanismus der homologen Rekombination (vgl. R.M. Torres, R. Kühn, Laboratory Protocols for Conditional Gene Targeting, Oxford University Press, 1997) in embryonalen Stammzellen (z.B. Maus 129/SV) bzw. die neue und offenbar sehr effiziente "nuclear transfer technology" (E. Pennisi, "After Dolly, a Pharming Frenzy", Science 279, S. 646-648, 1998) zu nennen. Die homologe Rekombination zwischen den in einem Chromosom vorhandenen DNA-Sequenzen und neuen, hinzugefügten clonierten DNA-Sequenzen ermöglicht das Einfügen eines klonierten Gens in das Genom einer lebenden Zelle anstelle des ursprünglichen Gens. Mit dieser Methode können bei Verwendung embryonaler Stammzellen via Chimären Tiere erhalten werden, die für das gewünschte Gen oder den gewünschten Genteil oder die gewünschte Mutation homozygot sind.

Ferner lassen sich durch Kreuzung des erfindungsgemäßen transgenen nicht menschlichen Säugers mit bereits etablierten transgenen nicht-melischlichen Säugern doppelt-transgene Säugetiermodelle herstellen.

Zur Herstellung eines erfindungsgemäßen transgenen nicht-mienschlichen Säugers über den Mechanismus des Genaustausches wird ein Gensubstitutionsvektor (z.B. auf der Basis des vorstehend beschriebenen Plasmids pCMV-PrPΔH1) und eine embryonale Stammzellinie des entsprechenden nicht-menschlichen Säugers (z. B. ist bei einer Maus ES 129/SV bevorzugt) verwendet. Die erhaltenen rekombinanten Klone der embryonalen Stammzellen werden dabei in Blastocysten injiziert und diese dann in geeignet vorbereitete weibliche Tiere zur Erzeugung von Chimären implantiert. "Geeignet vorbereitete weibliche Tiere" heißt dabei, daß die Tiere durch verschiedene Maßnahmen, z.B. durch eine Hormonbehandlung, auf ihre "austragende" Aufgabe vorbereitet worden sind. In diesem Zusammenhang sei auch auf "R.M. Torres, R. Kühn, s.o." verwiesen. Da es vorteilhaft ist, reinerbige-Tiere vorliegen zu haben, können die so erhaltenen heterozygoten Stämme dann mit einem anderen Tier des gleichen Stamms oder einem anderen (heterozygoten) Stamm verpaart werden. So kann durch Kreuzung ein homozygoter Zustand erreicht werden. Damit werden prionresistente Tiere hervorgebracht, die dann nach Standard-Verfahren weitergezüchtet werden können.

Die Gentherapie von Prionkrankheiten bei menschlichen oder tierischen Individuen kann durch Transfektion bzw. Transduktion von somatischen Zellen des betreffenden Organismus, wie z.B. Neuronen, Gliazellen, Fibroblasten oder Zellen der Hirnblutgefäße, mit dem erfindungsgemäßen Vektor erfolgen. Die Transduktion kann durch übliche Verfahren erfolgen. Liegt der Vektor als Virus-Partikel vor, ist es günstig, die Zellen mit diesen zu infizieren. Liegt er andererseits als Nukleinsäure, z.B. DNA, vor, ist es angeraten, die Zellen mit dieser zu transfizieren. Als Transfektionsfektionstechniken sind z.B. Elektroporation, CalciumPhosphat-Copräzipitation, Lipofektion und Partikel-Kanone zu nennen. Die Zellen können in dem Organismus vorliegen. Andererseits können die zu transduzierenden Zellen auch aus dem Organismus isoliert, außerhalb des Organismus transduziert und dann wieder in den Organismus zurückgeführt werden. Solche Zellen werden als autologe Zellen bezeichnet. Desweiteren können hinsichtlich des Organismus auch allogene Zellen zur Transduktion verwendet werden. Hierbei ist es günstig, wenn diese Zellen einem dem Organismus entsprechenden HLA-Typ angehören.

Ein weiterer Gegenstand der Erfindung ist ein Vakzinierungsmittel, das einen vorstehenden Vektor, ein vorstehendes Protein oder Fragmente davon und übliche Hilfsstoffe, wie Puffer, Verdünnungsmittel, Trägermittel etc. umfaßt.

Mit der vorliegenden Erfindung ist es möglich, im Sinne einer Prophylaxe das Angehen einer Prioninfektion zu unterbinden bzw. das Fortschreiten solcher Infektionen in bereits infizierten Individuen zu verhindern. Einerseits kann durch Herstellung transgener Nutztiere (z.B. Rinder, Schafe, Ziegen, Schweine, Hühner, Fische), die durch Tragen des mutierten PrP-Gens vor einer Prioninfektion von vornherein geschützt sind, das Risiko einer Ansteckung des Menschen über die Nahrungskette, über tierabgeleitete Arzneimittel bzw. Kosmetika eliminiert werden. Andererseits ermöglicht die vorliegende Erfindung, über eine in-vivo- bzw. ex-vivo-Gentherapie eine ansonsten tödliche Erkrankung zu verhindern bzw. zu therapieren.

Eine Gentherapie/-prophylaxe besitzt im Vergleich zu einer konventionellen Therapie verschiedene Vorteile, welche auf der Spezifität des molekularen Angriffspunktes (z.B. Eingriff in die Protein-Protein-Wechselwirkung zwischen PrP^{c} und PrP^{sc}) beruhen. Daraus resultiert auch die Nebenwirkungsfreiheit bei dennoch hoher Effizienz. Eine Gentherapie/-prophylaxe kann folgendermaßen vorgenommen werden:
1) Herstellung Prion-resistenter Nutztiere: Es werden mittels des erfindungsgemäßen Vektors transgene Tiere hergestellt, die statt des endogenen Wildtyp PrP^{c} oder zusätzlich zum endogenen Wildtyp-PrP^{c} ein mutiertes Prionprotein exprimieren, welches selbst nicht mehr in eine Protease-resistente PrP^{sc} Form überführt werden kann und zusätzlich die Konversion von Wildtyp-PrP^{c}, in PrP^{sc} verhindern kann. Im Vergleich zu transgenen "knockout" Tieren, d.h. Tieren, die überhaupt kein Prionprotein mehr exprimieren, haben die transgenen Tiere, die die mutierte Form exprimieren, den Vorteil, daß keine phänotypischen Unterschiede zu "Normaltieren" auftreten, da Tiere mit einem mutierten, nur in einer kleinen Region veränderten Prionprotein sicherlich noch die normalen physiologischen Funktionen des Prionproteins enthalten. Mittels dieser Genprophylaxe bei Nutztieren würde eine Ansteckung dieser transgenen Tiere und somit ein weiteres Ausbreiten z.B. der BSE-Epidemie, verhindert.
2) künstliche Insemination/in-vitro-Fertilisation: Sperma eines transgenen Prionresistenten Nutztieres, z.B. eines Rindes, wird verwendet, um auf dem heute in der Zucht von Nutztieren üblichen Weg der künstlichen Insemination weitere Generationen resistenter Nutztiere herzustellen. In bevorzugter Weise kommt auch eine in-vitro-Fertilisation in Frage, da diese den Vorteil bietet, die Embryonen noch vor ihrer Implantation in den Uterus auf ihren genetischen Status, insbesondere die Anwesenheit des Transgens, zu unteruchen.
3) ex-vivo Gentherapie: Autologe Zellen eines Patienten, welche z.B. in das ZNS implantierbar sind, werden entnommen und als Zellkultur mit dem erfindungsgemäßen Vektor transfiziert bzw. transduziert und in den Patienten reimplantiert.
4) in-vivo Gentherapie: Ein erfindungsgemäßer Vektor (z.B. ein rekombinantes Virus) mit einem offenen Leserahmen eines mutierten Prionproteins und geeigneten Kontrollelementen (z.B. Promotoren, Enhancer, Polyadenylierungssignal, usw.) wird verwendet, um direkt die betroffenen Zellen (z.B. Nervenzellen) zu infizieren und dadurch die Ausbreitung der Krankheit im ZNS zu verhindern.

Die Erfindung wird anhand der Figuren beschrieben, welche zeigen:

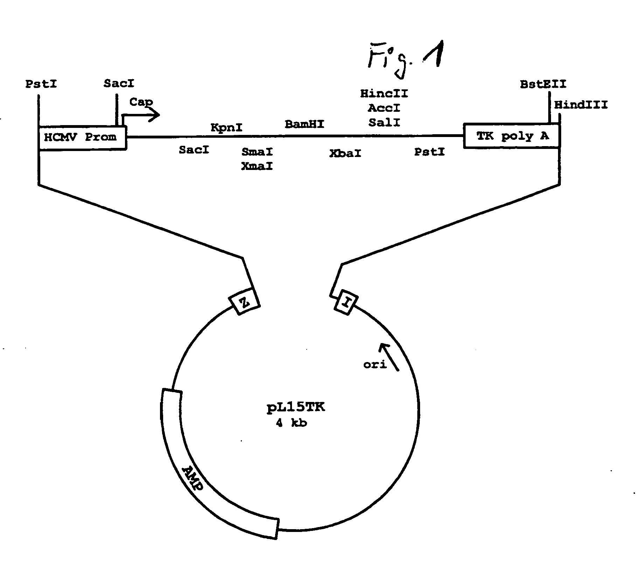

- Fig. 1:: Eukaryotischer Expressionsvektor pL1 5TK
Die verwendeten Abkürzungen haben folgende Bedeutungen:
HCMV Prom.: Menschlicher Cytomegalievirus-Promotor/Enhancer
AMP: Ampicillin-Resistenzgen
Z: Teil des Lac-Operons
I: Teil des Lac-Operons
(TK) Poly A: Polyadenylierungssignal des Herpes Simplex Virus Thymidinkinasegens
ori: Bakterieller Replikationsursprung
sowie die üblichen Abkürzungen für die Restriktionsschnittstellen
- Fig. 2:: Sequenzvergleich zwischen der DNA-Sequenz für Wildtyp-Maus-Prionprotein (Locht et al., Proc. Natl. Acad. Sci. USA 83, S. 6372 (1986); obere Reihe) und dem verwendeten Insert PrPΔH1 (Nukleotide 92 bis 867; untere Reihe) "Silent": stumme Mutation, die keinen Unterschied auf Aminosäureebene bedingt.
□ = Start- bzw. Stopcodon
- Fig. 3:: Western Blot nach transienter Transfektion von Mausneuroblastomzellen, die persistent mit Maus-Scarpie-Erregern infiziert worden sind, mit
Vektor (pL15tk) = Kontrolle (leerer Vektor)
pCMV-PrPΔH1 (erfindungsgemäß)
pCMV-PrP (Wildtyp)
- Fig. 4:: DNA-Sequenz des Inserts PrPΔH1
□ = Start- bzw. Stopcodon

Die Erfindung wird weiter durch die nachfolgenden Beispiele erläutert.

### Beispiel 1: Herstellung eines Vektors, der ein Insert für ein mutiertes Prion-Protein enthält

Die Herstellung des hinterlegten Vektors pCMV-PrPΔH1 erfolgte unter Verwendung des Expressionsvektors pL15TK. Dieser Vektor basiert auf pUC19 und enthält den humanen CMV-Promotor sowie das HSV-TK-Polyadenylierungssignal. Die Genkarte dieses Vektors ist in Fig. 1 gezeigt. Die Einklonierung des PrPΔH1-Inserts geschah dadurch, daß die überhängenden Enden ("sticky ends") des Inserts, welches mit den Restriktionsendonukleasen AatII und Sacl geschnitten worden war, mittels T4-DNA-Polymerase stumpf ("blunt") gemacht wurde und dann in die Smal-Schnittstelle von pL15TK einligiert wurden.

### Beispiel 2: Western Blot

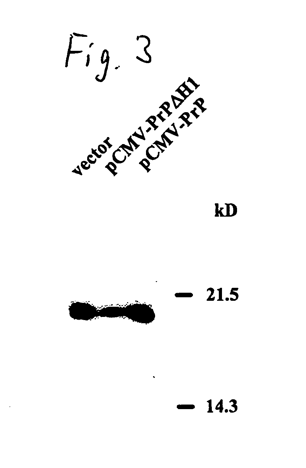

Das Plasmid pCMV-PrPΔH 1 wird in Maus-Neuroblastomzellen, die persistent mit dem Maus-Scrapie-Erreger infiziert sind (Race et al., Characterization of scrapie infection in mouse neuroblastoma cells, J. gen. Virol. 68, S. 1391-1399 (1987)), transfiziert. Nach ca. 48 Stunden werden die Zellen zur Herstellung cytoplasmatischer Extrakte lysiert und nach einem limitierten Proteinase K-Verdau, Guanidin-Isothiocyanat-Denaturierungund Verdau mit Peptid:N-Glycosidase F (PNGase F) im Western Blot analysiert. Das Ergebnis des Western Blot ist in Fig. 3 gezeigt. Dieser Western Blot belegt die Nichtkonvertierbarkeit der Mutante PrPΔH1 zu PrP^{sc} sowie ihre dominant negative Wirkung. Das Ausmaß der dominant-negativen Wirkung zeigt sich durch Vergleich der PrP^{sc}-Mengen, die gebildet wurden, wenn der Vektor pCMV-PrPΔH1 eingesetzt wurde (Fig. 3, Spur 2) mit Extrakten aus persistent infizierten Zellen, welche lediglich mit dem leeren Vektor transfiziert waren (Fig. 3, Spur 1).

## Patentansprüche

1. Vektor mit einem Nukleinsäureinsert, das für ein mutiertes Prionprotein kodiert, welches die Fähigkeit zur Konversion in die amyloidartige, krankheitsassoziierte Form des Prionproteins verloren hat und von dem eine Hemmwirkung auf die Konversion von in einem Organismus natürlicherweise vorhandenen endogenen Wildtyp-Prionproteins ausgeht, wobei das mutierte Prionprotein eine Deletion oder eine Substitution von mindestens zwei Aminosäuren zwischen den Aminosäuren 114 und 121, (H1-Bereich) aufweist.

2. Vektor nach Anspruch 1, **dadurch gekennzeichnet, dass** der Vektor ein Virus- oder ein Plasmid-Vektor ist.

3. Vektor nach Anspruch 1 oder 2, **dadurch gekennzeichnet, dass** der Virus-Vektor ein AAV-, Herpes Simplex Virus-, retroviraler, Adenovirus- oder Vacciniavirus-Vektor ist.

4. Mutiertes Prionprotein, welches die Fähigkeit zur Konversion in die amyloidartige, krankheitsassoziierte Form des Prionproteins verloren hat und von dem eine Hemmwirkung auf die Konversion von in einem Organismus natürlicherweise vorhandenen endogenen Wildtyp-Prionproteins ausgeht, wobei das mutierte Prionprotein eine Deletion oder eine Substitution von mindestens zwei Aminosäuren zwischen den Aminosäuren 114 und 121 (H1-Bereich) aufweist.

5. DNA kodierend für ein mutiertes Prionprotein gemäß Anspruch 4.

6. Transformierte Zelle enthaltend den Vektor nach einem der Ansprüche 1 bis 3.

7. Verfahren zur Herstellung des Proteins nach Anspruch 4, umfassend die Kultivierung der transformierten Zelle nach Anspruch 6 unter geeigneten Bedingungen.

8. Vakzinierungsmittel, enthaltend den Vektor nach einem der Ansprüche 1 bis 3 oder das Protein nach Anspruch. 4 bzw. Fragmente davon sowie übliche Hilfsstoffe.

9. Verwendung des Vektors nach einem der Ansprüche 1 bis 3 oder des Proteins nach Anspruch 4 oder der DNA nach Anspruch 5 zur Herstellung eines pharmazeutischen Zusammensetzung zur Therapie und/oder Prophylaxe von Prionkrankheiten.

10. Prion-resistenter nicht-menschlicher Säuger, **dadurch gekennzeichnet, dass** dieser ein mutiertes Prionprotein exprimiert, welches die Fähigkeit zur Konversion in die amyloidartige, krankheitsassozüerte Form des Prionproteins verloren hat und von dem eine Hemmwirkung auf die Konversion von in einem Organismus natürlicherweise vorhandenen endogenen Wildtyp-Prionproteins ausgeht, wobei das mutierte Prionprotein eine Deletion oder eine Substitution von mindestens zwei Aminosäuren zwischen den Aminosäuren 114 und 121 (H1-Bereich) aufweist.

11. Prion-resistenter nicht-menschlicher Säuger nach Anspruch 10, **dadurch gekennzeichnet, dass** dieser ein Nutztier ist.

12. Prion-resistenter nicht-menschlicher Säuger nach Anspruch 11, **dadurch gekennzeichnet, dass** das Nutztier ausgewählt ist aus Rindern, Schafen, Ziegen, Schweinen, Hühnern oder Fischen.

13. Prion-resistenter nicht-menschlicher Säuger nach einem der Ansprüche 10 bis 12, **dadurch gekennzeichnet, dass** dieser mittels Transgenese oder künstlicher Insemination oder in-vitro-Fertilisation erzeugt wird.

## Claims

1. A vector having a nucleic acid insert coding for a mutated prion protein, which has lost its capability of being convertible into the amyloid-like, disease-associated form of the prion protein and has an inhibitory effect on the conversion of the endogenous wild-type prion protein that is naturally present in the organism, wherein the mutated prion protein comprises a deletion or a substitution of at least two amino acids between the amino acids 114 and 121 (H1 region).

2. The vector according to claim 1, **characterised in that** the vector is a viral or plasmid vector.

3. The vector according to claim 1 or 2, **characterised in that** the viral vector is an AAV, herpes simplex virus, retrovirus, adenovirus or vaccinia virus vector.

4. A mutated prion protein, which has lost its capability of being convertible into the amyloid-like, disease-associated form of the prion protein and has an inhibitory effect on the conversion of the endogenous wild-type prion protein that is naturally present in the organism, wherein the mutated prion protein comprises a deletion or a substitution of at least two amino acids between the amino acids 114 and 121 (H1 region).

5. A DNA coding for a mutated prion protein according to claim 4.

6. A transformed cell comprising the vector according to any one of claims 1 to 3.

7. A process for the preparation of the protein according to claim 4, comprising the culturing of the transformed cell according to claim 6 under suitable conditions.

8. A vaccination agent, comprising the vector according to any one of claims 1 to 3 or the protein according to claim 4 and fragments thereof, respectively, as well as conventional auxiliary agents.

9. Use of the vector according to any of claims 1 to 3 or of the protein according to claim 4 or of the DNA according to claim 5 for the manufacture of a pharmaceutical composition for therapy and/or prophylaxis of prion diseases.

10. A prion-resistant non-human mammal, **characterised in that** it expresses a mutated prion protein, which has lost its capability of being convertible into the amyloid-like, disease-associated form of the prion protein and has an inhibitory effect on the conversion of the endogenous wild-type prion protein that is naturally present in the organism, wherein the mutated prion protein comprises a deletion or a substitution of at least two amino acids between the amino acids 114 and 121 (H 1 region).

11. The prion-resistant non-human mammal according to claim 10, **characterised in that** it is a working animal.

12. The prion-resistant non-human mammal according to claim 11, **characterised in that** the working animal is selected from cattle, sheep, goats, pigs, chickens or fish.

13. The prion-resistant non-human mammal according to any of claims 10 to 12, **characterised in that** it is produced by means of transgenesis or artificial insemination or in vitro fertilisation.

## Revendications

1. Vecteur avec un insert d'acide nucléique, qui code pour une protéine de prion mutée, laquelle a perdu l'aptitude à la conversion en la forme amyloïde associée à la maladie de la protéine de prion et de laquelle découle un effet d'inhibition sur la conversion d'une protéine de prion de type sauvage endogène naturellement présente dans un organisme, la protéine de prion mutée présentant une délétion ou une substitution d'au moins deux acides aminés entre les acides aminés 114 et 121, (domaine H1).

2. Vecteur selon la revendication 1, **caractérisé en ce que** le vecteur est un vecteur viral ou un plasmide.

3. Vecteur selon la revendication 1 ou 2, **caractérisé en ce que** le vecteur viral est un vecteur de virus AAV, de virus de l'herpès simplex, de virus rétroviral, de virus adénoviral ou de virus de la vaccine.

4. Protéine de prion mutée, qui a perdu l'aptitude à la conversion en la forme amyloïde associée à la maladie de la protéine de prion et de laquelle découle un effet d'inhibition sur la conversion d'une protéine de prion de type sauvage endogène naturellement présente dans un organisme, la protéine de prion mutée présentant une délétion ou une substitution d'au moins deux acides aminés entre les acides aminés 114 et 121 (domaine H1).

5. ADN codant pour une protéine de prion mutée selon la revendication 4.

6. Cellule transformée contenant le vecteur selon l'une des revendications 1 à 3.

7. Procédé de préparation de la protéine selon la revendication 4, contenant la culture de la cellule transformée selon la revendication 6 dans des conditions appropriées.

8. Moyen de vaccination, contenant le vecteur selon l'une des revendications 1 à 3 ou la protéine selon la revendication 4 ou ses fragments ainsi que des adjuvants usuels.

9. Utilisation du vecteur selon l'une des revendications 1 à 3 ou de la protéine selon la revendication 4 ou de l'ADN selon la revendication 5 pour la préparation d'une composition pharmaceutique destinée à la thérapie et/ou à la prophylaxie des maladies à prion.

10. Mammifère non humain prion-résistant, **caractérisé en ce que** celui-ci exprime une protéine de prion mutée, qui a perdu l'aptitude à la conversion en la forme amyloïde associée à la maladie de la protéine de prion, et de laquelle découle un effet d'inhibition sur la conversion d'une protéine de prion de type sauvage endogène naturellement présente dans un organisme, la protéine de prion mutée présentant une délétion ou une substitution d'au moins deux acides aminés entre les acides aminés 114 et 121 (domaine H1).

11. Mammifère non humain prion-résistant selon la revendication 10, **caractérisé en ce que** celui-ci est un animal destiné à la production.

12. Mammifère non humain prion-résistant selon la revendication 11, **caractérisé en ce que** l'animal destiné à la production est choisi parmi les bovins, ovins, caprins, porcidés, gallinacés ou les poissons.

13. Mammifère non humain prion-résistant selon l'une des revendications 10 à 12, **caractérisé en ce que** celui-ci est produit par transgenèse ou par insémination artificielle ou fécondation in-vitro.
